# EUROPEAN PATENT APPLICATION

(11) **EP 0 950 711 A2**
(43) Date of publication of application: **20.10.1999**
(21) Application number: 99200303.8
(22) Date of filing: 02.02.1999
(51) Int. Cl.: C12N 15/12, C07K 14/72, C12N 5/10, G01N 33/68

(54) **Gonadotropin receptor**

(30) Priority: 06.02.1998 EP 98200357; 27.07.1998 EP 98202519; 24.09.1998 EP 98203213
(71) Applicant: Akzo Nobel N.V., 6824 BM Arnhem (NL)
(72) Inventor: Van der Spek, Petrus Johannes, 5342 HM Oss (NL); Heikoop, Judith Christina, 5211 ND s'Hertogenbosch (NL)
(74) Representative: Kraak, Hajo

(57) **Abstract**

The present invention relates to newly identified DNA sequences which code for novel gonadotropin receptors, as well as to the complete genes and the encoded proteins. The invention furthermore relates to a method for identification of molecules able to bind to these receptors. The invention can be used for the development of new drugs for treatment of infertility or for contraception.

Sequence listing

(1) GENERAL INFORMATION:

(i) APPLICANT:

(A) NAME: Akzo Nobel N.V.

(B) STREET: Velperweg 76

(C) CITY: Arnhem

(E) COUNTRY: The Netherlands

(F) POSTAL CODE (ZIP): 6824 BM

(G) TELEPHONE: 0412 666379

(H) TELEFAX: 0412 650592

(ii) TITLE OF INVENTION: Novel gonadotropin receptor

(iii) NUMBER OF SEQUENCES: 16

(iv) COMPUTER READABLE FORM:

(A) MEDIUM TYPE: Floppy disk

(B) COMPUTER: IBM PC compatible

(C) OPERATING SYSTEM: PC-DOS/MS-DOS

(D) SOFTWARE: PatentIn Release #1.0, Version #1.30 (EPO)

(2) INFORMATION FOR SEQ ID NO: 1:

(i) SEQUENCE CHARACTERISTICS:

(A) LENGTH: 515 base pairs

(B) TYPE: nucleic acid

(C) STRANDEDNESS: single

(D) TOPOLOGY: linear

(ii) MOLECULE TYPE: cDNA

(xi) SEQUENCE DESCRIPTION: SEQ ID NO: 1:

GAACTCCTTC TGTTTCCTGG TCGTGGCCGG TGCCTACATC AAACTGTACT GTGACCTGCC 60

GCGGGGCGAC TTTGAGGCCG TGTGGGACTG CGCCATGGTG AGGCACGTGG CCTGGCTCAT 120

CTTCGCAGAC GGGCTCCTCT ACTGTCCCGT GGCCTTCCTC AGCTTTGCCT CCATGCTGGG 180

CCTCTTCCCT GTCACGCCCG AGGCCGTCAA GTCTGTCCTG CTGGTGGTGC TGCCCCTGCC 240

TGCCTGCCTC AACCCACTGC TGTACCTGCT CTTCAACCCC CACTTCCGGG ATGACCTTCG 300

GCGGCTTCGG CCCCGCGCAG GGGACTCAGG GCCCCTAGCC TATGCTGCGG CCGGGGAGCT 360

GGAGAAGAGC TCCTGTGATT CTACCCAGGC CCTGGTAGCC TTCTCTGATG TGGATCTCAT 420

TCTGGAAGCT TCTGAAGCTG GGCGGCCCCC TGGGCTGGAG ACCTATGGCT TCCCCTCAGT 480

GACCCTCATC TCCTGTCAGC AGCCAGGGGC CCCCA 515

(2) INFORMATION FOR SEQ ID NO: 2:

(i) SEQUENCE CHARACTERISTICS:

(A) LENGTH: 664 base pairs

(B) TYPE: nucleic acid

(C) STRANDEDNESS: single

(D) TOPOLOGY: linear

(ii) MOLECULE TYPE: cDNA

(xi) SEQUENCE DESCRIPTION: SEQ ID NO: 2:

GCAGTGGCTG CAAAGTAGCT GGGTTTCTTG CAGTTTTCTC CTCAGAAAGT GCCATATTTT 60

TATTAATGCT AGCAACTGTC GAAAGANGCT TATCTGCAAA AGATATAATG AAAAATGGGA 120

AGAGCAATCA TCTCAAACAG TTCCGGGTTG CTGCCCTTTT GGCTTTCCTA GGTGCTACAG 180

TAGCAGGCTG TTTTCCCCTT TTCCATAGAG GGGAATATTC TGCATCACCC CTTTGTTTGC 240

CATTTCCTAC AGGTGAAACG CCATCATTAG GATTCACTGT AACGTTAGTG NTATTAANCT 300

CACTAGCATT TTTATTAATG GCCGTTATCT ACACTAAGCT ATACTGCAAC TTGGAAAANG 360

AGGACCTCTC AGAAAACTCA CAATCTAGCA TGATTAAGCA TGTCGCTTGG CTAATCTTCA 420

CCAATTGCAT CTTTTTCTGC CCTGTGGCGT TTTTTTCATT TGCACCATTG ATCACTGCAA 480

TCTCTATCAG CCCCGAAATA ATGAAGTCTG TTACTCTGAT ATTTTTTCCA TTGCCTGCTT 540

GCCTGAATCC AGTCCTGTAT GTTTTCTTCA ACCCAAAGTT TAAAGAAGAC TGGAAGTTAC 600

TGAAGCGACG TGTTACCAAG AAAAGTGGAT CAGTTTCAGT TTCCATCAGT AGCCAAGGTG 660

GTTG

(2) INFORMATION FOR SEQ ID NO: 3:

(i) SEQUENCE CHARACTERISTICS:

(A) LENGTH: 606 base pairs

(B) TYPE: nucleic acid

(C) STRANDEDNESS: single

(D) TOPOLOGY: linear

(ii) MOLECULE TYPE: cDNA

(xi) SEQUENCE DESCRIPTION: SEQ ID NO: 3:

ATTGGAGTGT GGACCATAGC AGTTCTGGCA CTTACTTGTA ATGCTTTGGT GACTTCAACA 60

GTTTTCAGAT CCCCTCTGTA CATTTCCCCC ATTAAACTGT TAATTGGGGT CATCGCAGCA 120

GTGAACATGC TCACGGGAGT CTCCAGTGCC GTGCTGGCTG GTGTGGATGC GTTCACTTTT 180

GGCAGCTTTG CACGACATGG TGCCTGGTGG GAGAATGGGG TTGGTTGCCA TGTCATTGGT 240

TTTTTGTCCA TTTTTGCTTC AGAATCATCT GTTTTCCTGC TTACTCTGGC AGCCCTGGAG 300

CGTGGGTTCT CTGTGAAATA TTCTGCAAAA TTTGAAACGA AAGCTCCATT TTCTAGCCTG 360

AAAGTAATCA TTTTGCTCTG TGCCCTGCTG GCCTTGACCA TGGCCGCAGT TCCCCTGCTG 420

GGTGGCAGCA AGTATGGCGC CTCCCCTCTC TGCCTGCCTT TGCCTTTTGG GGAGCCCAGC 480

ACCATGGGCT ACATGGTCGC TCTCATCTTG CTCAATTCCC TTTGCTTCCT CATGATGACC 540

ATTGCCTACA CCAAGCTCTA CTGCAATTTG GACAAGGGAG ACCTGGAGAA TATTTGGGAC 600

TGCTCT 606

(2) INFORMATION FOR SEQ ID NO: 4:

(i) SEQUENCE CHARACTERISTICS:

(A) LENGTH: 171 amino acids

(B) TYPE: amino acid

(C) STRANDEDNESS: single

(D) TOPOLOGY: linear

(ii) MOLECULE TYPE: protein

(xi) SEQUENCE DESCRIPTION: SEQ ID NO: 4:

Asn Ser Phe Cys Phe Leu Val Val Ala Gly Ala Tyr Ile Lys Leu Tyr
1               5                   10                  15

Cys Asp Leu Pro Arg Gly Asp Phe Glu Ala Val Trp Asp Cys Ala Met
            20                  25                  30

Val Arg His Val Ala Trp Leu Ile Phe Ala Asp Gly Leu Leu Tyr Cys
        35                  40                  45

Pro Val Ala Phe Leu Ser Phe Ala Ser Met Leu Gly Leu Phe Pro Val
    50                  55                  60

Thr Pro Glu Ala Val Lys Ser Val Leu Leu Val Val Leu Pro Leu Pro
65                  70                  75                  80

Ala Cys Leu Asn Pro Leu Leu Tyr Leu Leu Phe Asn Pro His Phe Arg
                85                  90                  95

Asp Asp Leu Arg Arg Leu Arg Pro Arg Ala Gly Asp Ser Gly Pro Leu
            100                 105                 110

Ala Tyr Ala Ala Ala Gly Glu Leu Glu Lys Ser Ser Cys Asp Ser Thr
        115                 120                 125

Gln Ala Leu Val Ala Phe Ser Asp Val Asp Leu Ile Leu Glu Ala Ser
    130                 135                 140

Glu Ala Gly Arg Pro Pro Gly Leu Glu Thr Tyr Gly Phe Pro Ser Val
145                 150                 155                 160

Thr Leu Ile Ser Cys Gln Gln Pro Gly Ala Pro
                165                 170

(2) INFORMATION FOR SEQ ID NO: 5:

    (i) SEQUENCE CHARACTERISTICS:

        (A) LENGTH: 220 amino acids

        (B) TYPE: amino acid

        (C) STRANDEDNESS: single

        (D) TOPOLOGY: linear

   (ii) MOLECULE TYPE: protein

   (xi) SEQUENCE DESCRIPTION: SEQ ID NO: 5:

Ser Gly Cys Lys Val Ala Gly Phe Leu Ala Val Phe Ser Ser Glu Ser
1               5                   10                  15

Ala Ile Phe Leu Leu Met Leu Ala Thr Val Glu Arg Xaa Leu Ser Ala
            20                  25                  30

Lys Asp Ile Met Lys Asn Gly Lys Ser Asn His Leu Lys Gln Phe Arg
        35                  40                  45

Val Ala Ala Leu Leu Ala Phe Leu Gly Ala Thr Val Ala Gly Cys Phe
    50                  55                  60

Pro Leu Phe His Arg Gly Glu Tyr Ser Ala Ser Pro Leu Cys Leu Pro
65                  70                  75                  80

Phe Pro Thr Gly Glu Thr Pro Ser Leu Gly Phe Thr Val Thr Leu Val
                85                  90                  95

Xaa Leu Xaa Ser Leu Ala Phe Leu Leu Met Ala Val Ile Tyr Thr Lys
            100                 105                 110

Leu Tyr Cys Asn Leu Glu Xaa Glu Asp Leu Ser Glu Asn Ser Gln Ser
        115                 120                 125

Ser Met Ile Lys His Val Ala Trp Leu Ile Phe Thr Asn Cys Ile Phe
    130                 135                 140

Phe Cys Pro Val Ala Phe Phe Ser Phe Ala Pro Leu Ile Thr Ala Ile
145                 150                 155                 160

Ser Ile Ser Pro Glu Ile Met Lys Ser Val Thr Leu Ile Phe Phe Pro
                165                 170                 175

Leu Pro Ala Cys Leu Asn Pro Val Leu Tyr Val Phe Phe Asn Pro Lys
            180                 185                 190

Phe Lys Glu Asp Trp Lys Leu Leu Lys Arg Arg Val Thr Lys Lys Ser
        195                 200                 205

Gly Ser Val Ser Val Ser Ile Ser Ser Gln Gly Gly
    210                 215                 220

(2) INFORMATION FOR SEQ ID NO: 6:

(i) SEQUENCE CHARACTERISTICS:
(A) LENGTH: 202 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 6:

Ile Gly Val Trp Thr Ile Ala Val Leu Ala Leu Thr Cys Asn Ala Leu
1               5                   10                  15

Val Thr Ser Thr Val Phe Arg Ser Pro Leu Tyr Ile Ser Pro Ile Lys
            20                  25                  30

Leu Leu Ile Gly Val Ile Ala Ala Val Asn Met Leu Thr Gly Val Ser
        35                  40                  45
Ser Ala Val Leu Ala Gly Val Asp Ala Phe Thr Phe Gly Ser Phe Ala
    50                  55                  60
Arg His Gly Ala Trp Trp Glu Asn Gly Val Gly Cys His Val Ile Gly
65                  70                  75                  80
Phe Leu Ser Ile Phe Ala Ser Glu Ser Ser Val Phe Leu Leu Thr Leu
                85                  90                  95
Ala Ala Leu Glu Arg Gly Phe Ser Val Lys Tyr Ser Ala Lys Phe Glu
            100                 105                 110
Thr Lys Ala Pro Phe Ser Ser Leu Lys Val Ile Ile Leu Leu Cys Ala
        115                 120                 125
Leu Leu Ala Leu Thr Met Ala Ala Val Pro Leu Leu Gly Gly Ser Lys
    130                 135                 140
Tyr Gly Ala Ser Pro Leu Cys Leu Pro Leu Pro Phe Gly Glu Pro Ser
145                 150                 155                 160
Thr Met Gly Tyr Met Val Ala Leu Ile Leu Leu Asn Ser Leu Cys Phe
                165                 170                 175
Leu Met Met Thr Ile Ala Tyr Thr Lys Leu Tyr Cys Asn Leu Asp Lys
            180                 185                 190

Gly Asp Leu Glu Asn Ile Trp Asp Cys Ser
        195                 200

(2) INFORMATION FOR SEQ ID NO: 7:

(i) SEQUENCE CHARACTERISTICS:
(A) LENGTH: 1987 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 7:

CAAGGCCTCT GGGCAGTGGG AGGCTGAAGA CCTTCACCTT GATGATGAGG AGTCTTCAAA 60
AAGGCCCCTG GGCCTCCTTG CCAGACAAGC AGAGAACCAC TATGACCAGG ACCTGGATGA 120
GCTCCAGCTG GAGATGGAGG ACTCAAAGCC ACACCCCAGT GTCCAGTGTA GCCCTACTCC 180
AGGCCCCTTC AAGCCCTGTG AGTACCTCTT TGAAAGCTGG GGCATCCGCC TGGCCGTGTG 240
GGCCATCGTG TTGCTCTCCG TGCTCTGCAA TGGACTGGTG CTGCTGACCG TGTTCGCTGG 300
CGGGCCTGCC CCCCTGCCCC CGGTCAAGTT TGTGGTAGGT GCGATTGCAG GCGCCAACAC 360
CTTGACTGGC ATTTCCTGTG GCCTTCTAGC CTCAGTCGAT GCCCTGACCT TTGGTCAGTT 420
CTCTGAGTAC GGAGCCCGCT GGGAGACGGG GCTAGGCTGC CGGGCCACTG GCTTCCTGGC 480
AGTACTTGGG TCGGAGGCAT CGGTGCTGCT GCTCACTCTG GCCGCAGTGC AGTGCAGCGT 540
CTCCGTCTCC TGTGTCCGGG CCTATGGGAA GTCCCCCTCC CTGGGCAGCG TTCGAGCAGG 600
GGTCCTAGGC TGCCTGGCAC TGGCAGGGCT GGCCGCCGCA CTGCCCCTGG CCTCAGTGGG 660
AGAATACGGG GCCTCCCCAC TCTGCCTGCC CTACGCGCCA CCTGAGGGTC AGCCAGCAGC 720

CCTGGGCTTC ACCGTGGCCC TGGTGATGAT GAACTCCTTC TGTTTCCTGG TCGTGGCCGG 780

TGCCTACATC AAACTGTACT GTGACCTGCC GCGGGGCGAC TTTGAGGCCG TGTGGGACTG 840

CGCCATGGTG AGGCACGTGG CCTGGCTCAT CTTCGCAGAC GGGCTCCTCT ACTGTCCCGT 900

GGCCTTCCTC AGCTTCGCCT CCATGCTGGG CCTCTTCCCT GTCACGCCCG AGGCCGTCAA 960

GTCTGTCCTG CTGGTGGTGC TGCCCCTGCC TGCCTGCCTC AACCCACTGC TGTACCTGCT 1020

CTTCAACCCC CACTTCCGGG ATGACCTTCG GCGGCTTCGG CCCCGCGCAG GGGACTCAGG 1080

GCCCCTAGCC TATGCTGCGG CCGGGGAGCT GGAGAAGAGC TCCTGTGATT CTACCCAGGC 1140

CCTGGTAGCC TTCTCTGATG TGGATCTCAT TCTGGAAGCT TCTGAAGCTG GGCGGCCCCC 1200

TGGGCTGGAA CCTATGGCTC CCCTCAGGAC CCTCATTCCT GTCAGAGCCA GGGGCCCCCA 1260

GGCTGGAGGG CAGCCATTGT GTAGAGCCAG AGGGGAACCA CTTTGGGAAC CCCCAACCCT 1320

CCATGGATGG AGAACTGCTG CTGAGGGCAG AGGGATCTAC GCCAGCAGGT GGAGGCTTGT 1380

CAGGGGGTGG CGCTTTCAGC CCTCTGGCTT GGCCTTTGCT TCACACGTGT AAATATCCCT 1440

CCCCATTCTT CTCTTCCCCT CTCTTCCCTT TCCTCTCTCC CCCTCGGTGA ATGATGGCTG 1500

CTTCTAAAAC AAATACAACC AAAACTCAGC AGTGTGATCT ATAGCAGGAT GGCCCAGTAC 1560

CTGGCTCCAC TGATCACCTC TCTCCTGTGA CCATCACCAA CGGGTGCCCT CTTGGCCTGG 1620

CTTTCCCTTG GCCTTCCTCA GCTTCACCTT GATACTGGGC CTCTTCCTTG TCATGTCTGA 1680

AGCTGTGGAC CAGAGACCTG GACTTTTGTC TGCTTAAGGG AAATGAGGGA AGTAAAGACA 1740

GTGAAGGGGT GGAGGGTTGA TCAGGGCACA GTGGACAGGG AGACCTCACA GAGAAAGGCC 1800

TGGAAGGTGA TTTCCCGTGT GACTCATGGA TAGGATACAA AATGTGTTCC ATGTACCATT 1860

AATCTTGACA TATGCCATGC ATAAAGACTT CCTATTAAAA TAAGCTTTGG AAGAGATTAC 1920

ACATGATGTC TTTTTCTTAG AGATTCACAG TGCATGTTAG TGTAATAAAG AGATAAGTCC 1980

TACAGTA 2000

(2) INFORMATION FOR SEQ ID NO: 8:

(i) SEQUENCE CHARACTERISTICS:

(A) LENGTH: 497 amino acids

(B) TYPE: amino acid

(C) STRANDEDNESS: single

(D) TOPOLOGY: linear

(ii) MOLECULE TYPE: protein

(v) FRAGMENT TYPE: C-terminal

(xi) SEQUENCE DESCRIPTION: SEQ ID NO: 8:

Lys Ala Ser Gly Gln Trp Glu Ala Glu Asp Leu His Leu Asp Asp Glu
1               5                   10                  15

Glu Ser Ser Lys Arg Pro Leu Gly Leu Leu Ala Arg Gln Ala Glu Asn
            20                  25                  30

His Tyr Asp Gln Asp Leu Asp Glu Leu Gln Leu Glu Met Glu Asp Ser
        35                  40                  45

Lys Pro His Pro Ser Val Gln Cys Ser Pro Thr Pro Gly Pro Phe Lys
    50                  55                  60

Pro Cys Glu Tyr Leu Phe Glu Ser Trp Gly Ile Arg Leu Ala Val Trp
65                  70                  75                  80

Ala Ile Val Leu Leu Ser Val Leu Cys Asn Gly Leu Val Leu Leu Thr
                85                  90                  95

Val Phe Ala Gly Gly Pro Ala Pro Leu Pro Pro Val Lys Phe Val Val
            100                 105                 110

Gly Ala Ile Ala Gly Ala Asn Thr Leu Thr Gly Ile Ser Cys Gly Leu
        115                 120                 125
Leu Ala Ser Val Asp Ala Leu Thr Phe Gly Gln Phe Ser Glu Tyr Gly
    130                 135                 140
Ala Arg Trp Glu Thr Gly Leu Gly Cys Arg Ala Thr Gly Phe Leu Ala
145                 150                 155                 160
Val Leu Gly Ser Glu Ala Ser Val Leu Leu Leu Thr Leu Ala Ala Val
                165                 170                 175
Gln Cys Ser Val Ser Val Ser Cys Val Arg Ala Tyr Gly Lys Ser Pro
            180                 185                 190
Ser Leu Gly Ser Val Arg Ala Gly Val Leu Gly Cys Leu Ala Leu Ala
        195                 200                 205
Gly Leu Ala Ala Ala Leu Pro Leu Ala Ser Val Gly Glu Tyr Gly Ala
    210                 215                 220
Ser Pro Leu Cys Leu Pro Tyr Ala Pro Pro Glu Gly Gln Pro Ala Ala
225                 230                 235                 240
Leu Gly Phe Thr Val Ala Leu Val Met Met Asn Ser Phe Cys Phe Leu
                245                 250                 255
Val Val Ala Gly Ala Tyr Ile Lys Leu Tyr Cys Asp Leu Pro Arg Gly
            260                 265                 270

Asp Phe Glu Ala Val Trp Asp Cys Ala Met Val Arg His Val Ala Trp
        275                 280                 285

Leu Ile Phe Ala Asp Gly Leu Leu Tyr Cys Pro Val Ala Phe Leu Ser
    290                 295                 300

Phe Ala Ser Met Leu Gly Leu Phe Pro Val Thr Pro Glu Ala Val Lys
305                 310                 315                 320

Ser Val Leu Leu Val Val Leu Pro Leu Pro Ala Cys Leu Asn Pro Leu
                325                 330                 335

Leu Tyr Leu Leu Phe Asn Pro His Phe Arg Asp Asp Leu Arg Arg Leu
            340                 345                 350

Arg Pro Arg Ala Gly Asp Ser Gly Pro Leu Ala Tyr Ala Ala Ala Gly
        355                 360                 365

Glu Leu Glu Lys Ser Ser Cys Asp Ser Thr Gln Ala Leu Val Ala Phe
    370                 375                 380

Ser Asp Val Asp Leu Ile Leu Glu Ala Ser Glu Ala Gly Arg Pro Pro
385                 390                 395                 400

Gly Leu Glu Thr Tyr Gly Phe Pro Ser Val Thr Leu Ile Ser Cys Gln
                405                 410                 415

Gln Pro Gly Ala Pro Arg Leu Glu Gly Ser His Cys Val Glu Pro Glu
            420                 425                 430

Gly Asn His Phe Gly Asn Pro Gln Pro Ser Met Asp Gly Glu Leu Leu
        435                 440                 445

Leu Arg Ala Glu Gly Ser Thr Pro Ala Gly Gly Gly Leu Ser Gly Gly
    450                 455                 460

Gly Ala Phe Ser Pro Leu Ala Trp Pro Leu Leu His Thr Cys Lys Tyr
465                 470                 475                 480

Pro Ser Pro Phe Phe Ser Ser Pro Leu Phe Pro Phe Leu Ser Pro Pro
                485                 490                 495

Arg

2) INFORMATION FOR SEQ ID NO: 9:

(i) SEQUENCE CHARACTERISTICS:
(A) LENGTH: 3041 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 9:

ATGCGCTTGG AGGGAGAGGG CCGCTCAGCG AGGGCGGGAC AGAATCTCTC CCGGGCTGGG 60

AGTGCACGGC GCGGTGCGCC CAGGGACCTC AGCATGAACA ACCTCACAGA GCTTCAGCCT 120
GGCCTCTTCC ACCACCTGCG CTTCTTGGAG GAGCTGCGTC TCTCTGGGAA CCATCTCTCA 180
CACATCCCAG GACAAGCATT CTCTGGTCTC TACAGCCTGA AAATCCTGAT GCTGCAGAAC 240
AATCAGCTGG GAGGAATCCC CGCAGAGGCG CTGTGGGAGC TGCCGAGCCT GCAGTCGCTA 300
GACCTGAATT ATAACAAGCT GCAGGAGTTC CCTGTGGCCA TCCGGACCCT GGGCAGACTG 360
CAGGAACTGG GGTTCCATAA CAACAACATC AAGGCCATCC CAGAAAAGGC CTTCATGGGG 420
AACCCTCTGC TACAGACGAT ACACTTTTAT GATAACCCAA TCCAGTTTGT GGGAAGATCG 480
GCATTCCAGT ACCTGCCTAA ACTCCACACA CTATCTCTGA ATGGTGCCAT GGACATCCAG 540
GAGTTTCCAG ATCTCAAAGG CACCACCAGC CTGGAGATCC TGACCCTGAC CCGCGCAGGC 600
ATCCGGCTGC TCCCATCGGG GATGTGCCAA CAGCTGCCCA GGCTCCGAGT CCTGGAACTG 660
TCTCACAATC AAATTGAGGA GCTGCCCAGC CTGCACAGGT GTCAGAAATT GGAGGAAATC 720
GGCCTCCAAC ACAACCGCAT CTGGGAAATT GGAGCTGACA CCTTCAGCCA GCTGAGCTCC 780
CTGCAAGCCC TGGATCTTAG CTGGAACGCC ATCCGGTCCA TCCACCCCGA GGCCTTCTCC 840
ACCCTGCACT CCCTGGTCAA GCTGGACCTG ACAGACAACC AGCTGACCAC ACTGCCCCTG 900
GCTGGACTTG GGGGCTTGAT GCATCTGAAG CTCAAAGGGA ACCTTGCTCT CTCCCAGGCC 960
TTCTCCAAGG ACAGTTTCCC AAAACTGAGG ATCCTGGAGG TGCCTTATGC CTACCAGTGC 1020
TGTCCCTATG GGATGTGTGC CAGCTTCTTC AAGGCCTCTG GGCAGTGGGA GGCTGAAGAC 1080
CTTCACCTTG ATGATGAGGA GTCTTCAAAA AGGCCCCTGG GCCTCCTTGC CAGACAAGCA 1140
GAGAACCACT ATGACCAGGA CCTGGATGAG CTCCAGCTGG AGATGGAGGA CTCAAAGCCA 1200
CACCCCAGTG TCCAGTGTAG CCCTACTCCA GGCCCCTTCA AGCCCTGTGA GTACCTCTTT 1260
GAAAGCTGGG GCATCCGCCT GGCCGTGTGG GCCATCGTGT TGCTCTCCGT GCTCTGCAAT 1320
GGACTGGTGC TGCTGACCGT GTTCGCTGGC GGGCCTGCCC CCCTGCCCCC GGTCAAGTTT 1380
GTGGTAGGTG CGATTGCAGG CGCCAACACC TTGACTGGCA TTTCCTGTGG CCTTCTAGCC 1440
TCAGTCGATG CCCTGACCTT TGGTCAGTTC TCTGAGTACG GAGCCCGCTG GGAGACGGGG 1500
CTAGGCTGCC GGGCCACTGG CTTCCTGGCA GTACTTGGGT CGGAGGCATC GGTGCTGCTG 1560
CTCACTCTGG CCGCAGTGCA GTGCAGCGTC TCCGTCTCCT GTGTCCGGGC CTATGGGAAG 1620
TCCCCCTCCC TGGGCAGCGT TCGAGCAGGG GTCCTAGGCT GCCTGGCACT GGCAGGGCTG 1680
GCCGCCGCAC TGCCCCTGGC CTCAGTGGGA GAATACGGGG CCTCCCCACT CTGCCTGCCC 1740
TACGCGCCAC CTGAGGGTCA GCCAGCAGCC CTGGGCTTCA CCGTGGCCCT GGTGATGATG 1800
AACTCCTTCT GTTTCCTGGT CGTGGCCGGT GCCTACATCA AACTGTACTG TGACCTGCCG 1860
CGGGGCGACT TTGAGGCCGT GTGGGACTGC GCCATGGTGA GGCACGTGGC CTGGCTCATC 1920
TTCGCAGACG GGCTCCTCTA CTGTCCCGTG GCCTTCCTCA GCTTCGCCTC CATGCTGGGC 1980
CTCTTCCCTG TCACGCCCGA GGCCGTCAAG TCTGTCCTGC TGGTGGTGCT GCCCCTGCCT 2040
GCCTGCCTCA ACCCACTGCT GTACCTGCTC TTCAACCCCC ACTTCCGGGA TGACCTTCGG 2100

CGGCTTCGGC CCCGCGCAGG GGACTCAGGG CCCCTAGCCT ATGCTGCGGC CGGGGAGCTG 2160
GAGAAGAGCT CCTGTGATTC TACCCAGGCC CTGGTAGCCT TCTCTGATGT GGATCTCATT 2220
CTGGAAGCTT CTGAAGCTGG GCGGCCCCCT GGGCTGGAGA CCTATGGCTT CCCCTCAGTG 2280
ACCCTCATCT CCTGTCAGCA GCCAGGGGCC CCCAGGCTGG AGGGCAGCCA TTGTGTAGAG 2340
CCAGAGGGGA ACCACTTTGG GAACCCCCAA CCCTCCATGG ATGGAGAACT GCTGCTGAGG 2400
GCAGAGGGAT CTACGCCAGC AGGTGGAGGC TTGTCAGGGG GTGGCGCTTT CAGCCCTCTG 2460
GCTTGGCCTT TGCTTCACAC GTGTAAATAT CCCTCCCCAT TCTTCTCTTC CCCTCTCTTC 2520
CCTTTCCTCT CTCCCCCTCG GTGAATGATG GCTGCTTCTA AAACAAATAC AACCAAAACT 2580
CAGCAGTGTG ATCTATAGCA GGATGGCCCA GTACCTGGCT CCACTGATCA CCTCTCTCCT 2640
GTGACCATCA CCAACGGGTG CCCTCTTGGC CTGGCTTTCC CTTGGCCTTC CTCAGCTTCA 2700
CCTTGATACT GGGCCTCTTC CTTGTCATGT CTGAAGCTGT GGACCAGAGA CCTGGACTTT 2760
TGTCTGCTTA AGGGAAATGA GGGAAGTAAA GACAGTGAAG GGGTGGAGGG TTGATCAGGG 2820
CACAGTGGAC AGGGAGACCT CACAGAGAAA GGCCTGGAAG GTGATTTCCC GTGTGACTCA 2880
TGGATAGGAT ACAAAATGTG TTCCATGTAC CATTAATCTT GACATATGCC ATGCATAAAG 2940
ACTTCCTATT AAAATAAGCT TTGGAAGAGA TTACACATGA TGTCTTTTTC TTAGAGATTC 3000
ACAGTGCATG TTAGTGTAAT AAAGAGATAA GTCCTACAGT A 3041

(2) INFORMATION FOR SEQ ID NO: 10:

(i) SEQUENCE CHARACTERISTICS:
(A) LENGTH: 847 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 10:

Met Arg Leu Glu Gly Glu Gly Arg Ser Ala Arg Ala Gly Gln Asn Leu
1               5                   10                  15

Ser Arg Ala Gly Ser Ala Arg Arg Gly Ala Pro Arg Asp Leu Ser Met
            20                  25                  30

Asn Asn Leu Thr Glu Leu Gln Pro Gly Leu Phe His His Leu Arg Phe
        35                  40                  45

Leu Glu Glu Leu Arg Leu Ser Gly Asn His Leu Ser His Ile Pro Gly
    50                  55                  60

Gln Ala Phe Ser Gly Leu Tyr Ser Leu Lys Ile Leu Met Leu Gln Asn
65                  70                  75                  80

Asn Gln Leu Gly Gly Ile Pro Ala Glu Ala Leu Trp Glu Leu Pro Ser
                85                  90                  95

Leu Gln Ser Leu Asp Leu Asn Tyr Asn Lys Leu Gln Glu Phe Pro Val
            100                 105                 110

Ala Ile Arg Thr Leu Gly Arg Leu Gln Glu Leu Gly Phe His Asn Asn
        115                 120                 125

Asn Ile Lys Ala Ile Pro Glu Lys Ala Phe Met Gly Asn Pro Leu Leu
    130                 135                 140

Gln Thr Ile His Phe Tyr Asp Asn Pro Ile Gln Phe Val Gly Arg Ser
145                 150                 155                 160

Ala Phe Gln Tyr Leu Pro Lys Leu His Thr Leu Ser Leu Asn Gly Ala
                165                 170                 175

Met Asp Ile Gln Glu Phe Pro Asp Leu Lys Gly Thr Thr Ser Leu Glu
            180                 185                 190
Ile Leu Thr Leu Thr Arg Ala Gly Ile Arg Leu Leu Pro Ser Gly Met
        195                 200                 205
Cys Gln Gln Leu Pro Arg Leu Arg Val Leu Glu Leu Ser His Asn Gln
    210                 215                 220
Ile Glu Glu Leu Pro Ser Leu His Arg Cys Gln Lys Leu Glu Glu Ile
225                 230                 235                 240
Gly Leu Gln His Asn Arg Ile Trp Glu Ile Gly Ala Asp Thr Phe Ser
                245                 250                 255
Gln Leu Ser Ser Leu Gln Ala Leu Asp Leu Ser Trp Asn Ala Ile Arg
            260                 265                 270
Ser Ile His Pro Glu Ala Phe Ser Thr Leu His Ser Leu Val Lys Leu
        275                 280                 285
Asp Leu Thr Asp Asn Gln Leu Thr Thr Leu Pro Leu Ala Gly Leu Gly
    290                 295                 300
Gly Leu Met His Leu Lys Leu Lys Gly Asn Leu Ala Leu Ser Gln Ala
305                 310                 315                 320
Phe Ser Lys Asp Ser Phe Pro Lys Leu Arg Ile Leu Glu Val Pro Tyr
                325                 330                 335
Ala Tyr Gln Cys Cys Pro Tyr Gly Met Cys Ala Ser Phe Phe Lys Ala
            340                 345                 350

Ser Gly Gln Trp Glu Ala Glu Asp Leu His Leu Asp Asp Glu Glu Ser
        355                 360                 365

Ser Lys Arg Pro Leu Gly Leu Leu Ala Arg Gln Ala Glu Asn His Tyr
    370                 375                 380

Asp Gln Asp Leu Asp Glu Leu Gln Leu Glu Met Glu Asp Ser Lys Pro
385                 390                 395                 400

His Pro Ser Val Gln Cys Ser Pro Thr Pro Gly Pro Phe Lys Pro Cys
                405                 410                 415

Glu Tyr Leu Phe Glu Ser Trp Gly Ile Arg Leu Ala Val Trp Ala Ile
            420                 425                 430

Val Leu Leu Ser Val Leu Cys Asn Gly Leu Val Leu Leu Thr Val Phe
        435                 440                 445

Ala Gly Gly Pro Ala Pro Leu Pro Pro Val Lys Phe Val Val Gly Ala
    450                 455                 460

Ile Ala Gly Ala Asn Thr Leu Thr Gly Ile Ser Cys Gly Leu Leu Ala
465                 470                 475                 480

Ser Val Asp Ala Leu Thr Phe Gly Gln Phe Ser Glu Tyr Gly Ala Arg
                485                 490                 495

Trp Glu Thr Gly Leu Gly Cys Arg Ala Thr Gly Phe Leu Ala Val Leu
            500                 505                 510

Gly Ser Glu Ala Ser Val Leu Leu Leu Thr Leu Ala Ala Val Gln Cys
        515                 520                 525

Ser Val Ser Val Ser Cys Val Arg Ala Tyr Gly Lys Ser Pro Ser Leu
    530                 535                 540
Gly Ser Val Arg Ala Gly Val Leu Gly Cys Leu Ala Leu Ala Gly Leu
545                 550                 555                 560
Ala Ala Ala Leu Pro Leu Ala Ser Val Gly Glu Tyr Gly Ala Ser Pro
                565                 570                 575
Leu Cys Leu Pro Tyr Ala Pro Pro Glu Gly Gln Pro Ala Ala Leu Gly
            580                 585                 590
Phe Thr Val Ala Leu Val Met Met Asn Ser Phe Cys Phe Leu Val Val
        595                 600                 605
Ala Gly Ala Tyr Ile Lys Leu Tyr Cys Asp Leu Pro Arg Gly Asp Phe
    610                 615                 620
Glu Ala Val Trp Asp Cys Ala Met Val Arg His Val Ala Trp Leu Ile
625                 630                 635                 640
Phe Ala Asp Gly Leu Leu Tyr Cys Pro Val Ala Phe Leu Ser Phe Ala
                645                 650                 655
Ser Met Leu Gly Leu Phe Pro Val Thr Pro Glu Ala Val Lys Ser Val
            660                 665                 670
Leu Leu Val Val Leu Pro Leu Pro Ala Cys Leu Asn Pro Leu Leu Tyr
        675                 680                 685
Leu Leu Phe Asn Pro His Phe Arg Asp Asp Leu Arg Arg Leu Arg Pro
    690                 695                 700

Arg Ala Gly Asp Ser Gly Pro Leu Ala Tyr Ala Ala Ala Gly Glu Leu
705                 710                 715                 720

Glu Lys Ser Ser Cys Asp Ser Thr Gln Ala Leu Val Ala Phe Ser Asp
                725                 730                 735

Val Asp Leu Ile Leu Glu Ala Ser Glu Ala Gly Arg Pro Pro Gly Leu
            740                 745                 750

Glu Thr Tyr Gly Phe Pro Ser Val Thr Leu Ile Ser Cys Gln Gln Pro
        755                 760                 765

Gly Ala Pro Arg Leu Glu Gly Ser His Cys Val Glu Pro Glu Gly Asn
    770                 775                 780

His Phe Gly Asn Pro Gln Pro Ser Met Asp Gly Glu Leu Leu Leu Arg
785                 790                 795                 800

Ala Glu Gly Ser Thr Pro Ala Gly Gly Gly Leu Ser Gly Gly Gly Ala
                805                 810                 815

Phe Ser Pro Leu Ala Trp Pro Leu Leu His Thr Cys Lys Tyr Pro Ser
            820                 825                 830

Pro Phe Phe Ser Ser Pro Leu Phe Pro Phe Leu Ser Pro Pro Arg
        835                 840                 845

(2) INFORMATION FOR SEQ ID NO: 11:

(i) SEQUENCE CHARACTERISTICS:

(A) LENGTH: 279 base pairs

(B) TYPE: nucleic acid

(C) STRANDEDNESS: single
(D) TOPOLOGY: linear

(ii) MOLECULE TYPE: cDNA

(xi) SEQUENCE DESCRIPTION: SEQ ID NO: 11:

TCCAGAATAA TCAGTTGAAA ACAGTACCCA GTGAAGCCAT TCGAGGGCTG AGTGCTTTGC 60
AGTCTTTGNG TTTAGATGCC AACCATATTA CCTNAGTCCC CGAGGACAGT TTTGAAGGAC 120
TTGTTCAGTT ACGGCATCTG TGGCTGGATG ACAACAGCTT GACGGAGGTG CCTGTGCACC 180
NCCTCAGCAA TCTGCCCACC CTACAGGCGC TGANCCTGGC TCTCAACAAG ATCTCAAGNA 240
TCCCTGACTT TGCATTTACC AACCTTTCAA GCCTGGTAG 279

(2) INFORMATION FOR SEQ ID NO: 12:

(i) SEQUENCE CHARACTERISTICS:
(A) LENGTH: 284 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 12:

CAACAACATC AAGGCCATCC CAGAAAAGGC CTTCATGGGG AACCCTCTGC TACAGACGAT 60
ACACTTTTAT GATAACCCAA TCCAGTTTGT GGGAAGATCG GCATTCCAGT ACCTGCCTAA 120
ACTCCACACA CTATCTCTGA ATGGTGCCAT GGACATCCAG GAGTTTCCAG ATCTCAAAGG 180

CACCACCAGC CTGGAGATCC TGACCCTGAC CCGCGCAGGC ATCCGGCTGC TCCCATCGGG 240

GATGTGCCAA CAGCTGCCCA GGCTCCGAGT CCTGTGAGTG CNAA 284

(2) INFORMATION FOR SEQ ID NO: 13:

(i) SEQUENCE CHARACTERISTICS:

(A) LENGTH: 764 base pairs

(B) TYPE: nucleic acid

(C) STRANDEDNESS: single

(D) TOPOLOGY: linear

(ii) MOLECULE TYPE: cDNA

(xi) SEQUENCE DESCRIPTION: SEQ ID NO: 13:

GCAGTGGCTG CAAAGTAGCT GGGTTTCTTG CAGTTTTCTC CTCAGAAAGT GCCATATTTT 60

TATTAATGCT AGCAACTGTC GAAAGANGCT TATCTGCAAA AGATATAATG AAAAATGGGA 120

AGAGCAATCA TCTCAAACAG TTCCGGGTTG CTGCCCTTTT GGCTTTCCTA GGTGCTACAG 180

TAGCAGGCTG TTTTCCCCTT TTCCATAGAG GGGAATATTC TGCATCACCC CTTTGTTTGC 240

CATTTCCTAC AGGTGAAACG CCATCATTAG GATTCACTGT AACGTTAGTG NTATTAANCT 300

CACTAGCATT TTTATTAATG GCCGTTATCT ACACTAAGCT ATACTGCAAC TTGGAAAANG 360

AGGACCTCTC AGAAAACTCA CAATCTAGCA TGATTAAGCA TGTCGCTTGG CTAATCTTCA 420

CCAATTGCAT CTTTTTCTGC CCTGTGGCGT TTTTTTCATT TGCACCATTG ATCACTGCAA 480

TCTCTATCAG CCCCGAAATA ATGAAGTCTG TTACTCTGAT ATTTTTTCCA TTGCCTGCTT 540

GCCTGAATCC AGTCCTGTAT GTTTTCTTCA ACCCAAAGTT TAAAGAAGAC TGGAAGTTAC 600

TGAAGCGACG TGTTACCAAG AAAAGTGGAT CAGTTTCAGT TTCCATCAGT AGCCAAGGTG 660

GTTGTCTGGA ACAGGATTTC TACTACGACT GTGGCATGTA CTCACATTTG CAGGGCAACC 720

TGACTGTTTG CGACTGCTGC GAATCGTTTC TTTTAACAAA GCCA 764

(2) INFORMATION FOR SEQ ID NO: 14:

(i) SEQUENCE CHARACTERISTICS:

(A) LENGTH: 92 amino acids

(B) TYPE: amino acid

(C) STRANDEDNESS: single

(D) TOPOLOGY: linear

(ii) MOLECULE TYPE: protein

(v) FRAGMENT TYPE: internal

(ix) FEATURE:

(A) NAME/KEY: Protein

(B) LOCATION:1..277

(xi) SEQUENCE DESCRIPTION: SEQ ID NO: 14:

Gln Asn Asn Gln Leu Lys Thr Val Pro Ser Glu Ala Ile Arg Gly Leu
1 5 10

Ser Ala Leu Gln Ser Leu Xaa Leu Asp Ala Asn His Ile Thr Xaa Val
15 20 25 30

Pro Glu Asp Ser Phe Glu Gly Leu Val Gln Leu Arg His Leu Trp Leu
        35                  40                  45

Asp Asp Asn Ser Leu Thr Glu Val Pro Val His Xaa Leu Ser Asn Leu
    50                  55                  60

Pro Thr Leu Gln Ala Leu Xaa Leu Ala Leu Asn Lys Ile Ser Xaa Ile
65 70 75

Pro Asp Phe Ala Phe Thr Asn Leu Ser Ser Leu Val
80                  85                  90

(2) INFORMATION FOR SEQ ID NO: 15:

(i) SEQUENCE CHARACTERISTICS:
(A) LENGTH: 91 amino acids
(B) TYPE: amino acid
(C) STRANDEDNESS: single
(D) TOPOLOGY: linear

(ii) MOLECULE TYPE: protein

(v) FRAGMENT TYPE: C-terminal

(ix) FEATURE:
(A) NAME/KEY: Protein
(B) LOCATION:1..273

(xi) SEQUENCE DESCRIPTION: SEQ ID NO: 15:

Asn Asn Ile Lys Ala Ile Pro Glu Lys Ala Phe Met Gly Asn Pro Leu
1               5                   10                  15

Leu Gln Thr Ile His Phe Tyr Asp Asn Pro Ile Gln Phe Val Gly Arg
            20                  25                  30

Ser Ala Phe Gln Tyr Leu Pro Lys Leu His Thr Leu Ser Leu Asn Gly
        35                  40                  45

Ala Met Asp Ile Gln Glu Phe Pro Asp Leu Lys Gly Thr Thr Ser Leu
    50                  55                  60

Glu Ile Leu Thr Leu Thr Arg Ala Gly Ile Arg Leu Leu Pro Ser Gly
65                    70                    75

Met Cys Gln Gln Leu Pro Arg Leu Arg Val Leu
80                    85                    90

(2) INFORMATION FOR SEQ ID NO: 16:

(i) SEQUENCE CHARACTERISTICS:
(A) LENGTH: 254 amino acids
(B) TYPE: amino acid
(C) STRANDEDNESS: single
(D) TOPOLOGY: linear

(ii) MOLECULE TYPE: protein

(v) FRAGMENT TYPE: internal

(ix) FEATURE:
(A) NAME/KEY: Protein
(B) LOCATION:1

(xi) SEQUENCE DESCRIPTION: SEQ ID NO: 16:

Ser Gly Cys Lys Val Ala Gly Phe Leu Ala Val Phe Ser Ser Glu Ser
        1                   5                   10

Ala Ile Phe Leu Leu Met Leu Ala Thr Val Glu Arg Xaa Leu Ser Ala
15                  20                  25                  30

Lys Asp Ile Met Lys Asn Gly Lys Ser Asn His Leu Lys Gln Phe Arg
        35                  40                  45

Val Ala Ala Leu Leu Ala Phe Leu Gly Ala Thr Val Ala Gly Cys Phe
    50                  55                  60

Pro Leu Phe His Arg Gly Glu Tyr Ser Ala Ser Pro Leu Cys Leu Pro
65 70 75

Phe Pro Thr Gly Glu Thr Pro Ser Leu Gly Phe Thr Val Thr Leu Val
80 85 90

Xaa Leu Xaa Ser Leu Ala Phe Leu Leu Met Ala Val Ile Tyr Thr Lys
95 100 105 110

Leu Tyr Cys Asn Leu Glu Xaa Glu Asp Leu Ser Glu Asn Ser Gln Ser
        115                 120                 125

Ser Met Ile Lys His Val Ala Trp Leu Ile Phe Thr Asn Cys Ile Phe
    130                 135                 140

Phe Cys Pro Val Ala Phe Phe Ser Phe Ala Pro Leu Ile Thr Ala Ile
145 150 155

Ser Ile Ser Pro Glu Ile Met Lys Ser Val Thr Leu Ile Phe Phe Pro
160 165 170

Leu Pro Ala Cys Leu Asn Pro Val Leu Tyr Val Phe Phe Asn Pro Lys
175 180 185 190

Phe Lys Glu Asp Trp Lys Leu Leu Lys Arg Arg Val Thr Lys Lys Ser
        195                 200                 205

Gly Ser Val Ser Val Ser Ile Ser Ser Gln Gly Gly Cys Leu Glu Gln
            210                 215                 220

Asp Phe Tyr Tyr Asp Cys Gly Met Tyr Ser His Leu Gln Gly Asn Leu
        225                 230                 235

Thr Val Cys Asp Cys Cys Glu Ser Phe Leu Leu Thr Lys Pro
    240                 245                 250

## Description

The present invention relates to newly identified DNA sequences which code for novel gonadotropin receptors, as well as to the complete genes and the encoded proteins. The invention furthermore relates to a method for identification of molecules able to bind to these receptors or specific domains thereof. The invention therefore has useful applications in fields including basic biomedical and biochemical research and drug development.

Gonadotropins act on specific gonadal cell types to initiate ovarian and testicular differentiation and steroidogenesis. The actions of these pituitary and placental hormones are mediated by specific plasma membrane receptors that are members of the large family of G-protein coupled receptors. They consist of a single polypeptide with seven transmembrane domains and are able to interact with the Gₛ protein, leading to the activation of adenyl cyclase.

Isolation of the FSH, LH and TSH receptor (hereafter called gonadotropin receptors) cDNAs indicates that these glycoprotein hormone receptors are homologous in their transmembrane regions, but have long and divergent extracellular domains resembling proteins of the leucine-rich protein family (Tilly et al, 1992, Endocrinol., *131*, 799-806; Parmentier et al, 1989, Science, *246*, 1620-1622; Jia et al, 1991, Mol. Endocrinol., *5*, 759-768).

There are several indications pointing to the presence, next to the FSH, LH and TSH receptor, of an additional glycoprotein receptor in the human body. Blithe et al, (1991, Endocrinol., *129*, 2257-2259) have suggested a specific role for α-hCG during pregnancy that can not be exerted by the hCG dimer, namely stimulation of prolactin (PRL) secretion from term pregnancy decidual cells. PRL appears to be involved in endometrial cell proliferation and attachment (Negami and Tominaga, 1991, Horm.Res.[Suppl.1], *35*, 50-57). Since the free α-subunit of hCG is not able to elicit a biological response via the LH/CG receptor, the involvement of a novel glycoprotein receptor is very likely. Moreover, *in vitro* the α-subunit acts synergistically with progesterone to induce decidualisation of human endometrial stromal cells (Moy et al, 1996, Endocrin., *137*, 1332-1339). Decidual cells are important for implantation and provide nutritional support for the embryo.

Furthermore, in GnRH-antagonist treated female cyclic rats hCG and LH preparations induce ovulation at a similar dose-level. However, in the hCG treated animals embryo implantation is negatively effected whereas, in contrast, LH treated animals show normal implantation. Preliminary experiments indicate that this different effect on implantation is only partly caused by the different half-life of hCG and LH. The uterus and the ovary, therefore, may contain LH/CG receptors with a different affinity for hCG and LH i.e. a novel glycoprotein receptor.

At present, however, only the nucleotide sequences of the human FSH, LH and TSH receptors have been elucidated and no direct data confirming the presence of additional gonadotropin receptors have been obtained. It will be clear that there is a great need for the elucidation of other receptors, in order to unravel the various roles these receptors play in normal physiology and pathology.

A better knowledge of these receptors, their mechanism of action and of the ligands which bind to these receptors might help to create a better insight in the underlying mechanism of the hormone signal transduction pathway, which eventually will lead to better treatment of fertility associated diseases and abnormalities linked to suboptimal hormone/receptor functioning.

The present invention provides for such novel receptors. More specific, the present invention provides for polynucleotide sequences comprising SEQ ID NO:1, SEQ ID NO:2, SEQ ID NO:3, SEQ ID NO:7, SEQ ID NO:9, SEQ ID NO:11, SEQ ID NO:12, SEQ ID NO:13 or nucleotides 2-1487 of SEQ ID NO:7 or nucleotides 1-2541 of SEQ ID NO:9.

The sequences of the present invention can be used as probes or as a source to prepare synthetic oligonucleotides to be used as primers in DNA amplification reactions allowing the isolation and identification of the complete gene. The complete genetic sequence can be used in the preparation of vector molecules for expression of the protein in suitable host cells.

Using the sequence information provided herein, complete genes or variants thereof can be derived from cDNA or genomic DNA from natural sources or synthesized using known methods.

Thus, an additional embodiment of the invention is a method to isolate a gene comprising the steps: a) hybridizing a DNA according to the present invention under stringent conditions against nucleic acids being RNA, (genomic) DNA or cDNA isolated preferably from tissues which highly express the DNA of interest; and b) isolating said nucleic acids by methods known to a skilled person in the art. The tissues preferably are from human origin. Preferably ribonucleic acids are isolated from reproductive tissues, preferably ovary, testis or uterus.

The hybridization conditions are preferably highly stringent.

According to the present invention the term "stringent" means washing conditions of 1 x SSC, 0.1% SDS at a temperature of 65 °C; highly stringent conditions refer to a reduction in SSC towards 0.3 x SSC.

Thus, the invention also includes the entire coding sequence part of which is indicated in SEQ ID NOs: 1, 2, 3, 7, 11, 12 or 13. A complete sequence is shown in SEQ ID NO:9. Furthermore, to accommodate codon variability, the invention also includes sequences coding for the same amino acid sequences as the sequences disclosed herein. Also portions of the coding sequences coding for individual domains of the expressed protein are part of the invention as well as allelic and species variations thereof. Sometimes, a gene is expressed in a certain tissue as a splicing variant, resulting in an altered 5' or 3' mRNA or the inclusion of an additional exon sequence. Alternatively, the messenger might have an exon less as compared to its counterpart as indicated in one of the sequences enlisted here. These sequences as well as the proteins encoded by these sequences all are expected to perform the same or similar functions and form also part of the invention.

The sequence information as provided herein should not be so narrowly construed as to require inclusion of erroneously identified bases. The specific sequence disclosed herein can be readily used to isolate the complete genes which in turn can easily be subjected to further sequence analyses thereby identifying sequencing errors.

Thus, in one aspect, the present invention provides for isolated polynucleotides encoding a novel gonadotropin receptor.

The DNA according to the invention may be obtained from cDNA. Alternatively, the coding sequence might be genomic DNA, or prepared using DNA synthesis techniques. The polynucleotide may also be in the from of RNA. If the polynucleotide is DNA, it may be in single stranded or double stranded form. The single strand might be the coding strand or the non-coding (anti-sense) strand.

The present invention further relates to polynucleotides which have at least 80%, preferably 90% and more preferably 95% and even more preferably at least 98% identity with SEQ ID NOs:1, 2, 3, 7, 9, 11, 12 or 13 or with nucleotides 2-1487 of SEQ ID NO:7 or 1-2541 of SEQ ID NO:9. Such polynucleotides encode polypeptides which retain the same biological function or activity as the natural, mature protein. Alternatively, also fragments of the above mentioned polynucleotides which code for domains of the receptor proteins which still are capable of binding to ligands are embodied in the invention.

The percentage of identity between two sequences can be determined with programs such as DNAMAN (Lynnon Biosoft, version 3.2). Using this program two sequences can be aligned using the optimal alignment algorithm of Smith and Waterman (1981, J. Mol. Biol, *147*:195-197). After alignment of the two sequences the percentage identity can be calculated by dividing the number of identical nucleotides between the two sequences by the length of the aligned sequences minus the length of all gaps.

The DNA according to the invention will be very useful for *in vivo* or *in vitro* expression of the novel receptor proteins according to the invention in sufficient quantities and in substantially pure form.

In another aspect of the invention, there is provided for a gonadotropin receptor comprising the amino acid sequence encoded by the above described DNA molecules.

Preferably, the gonadotropin receptor according to the invention comprises an amino acid sequence shown in SEQ ID NO:4, SEQ ID NO:5, SEQ ID NO:6, SEQ ID NO:8, SEQ ID NO:10, SEQ ID NO:14, SEQ ID NO:15 or SEQ ID NO:16.

The receptor according to the invention furthermore distinguishes itself from the other known gonadotropin receptors in differences in tissue distribution, indicating that there may be important differences between these tissues at the level of gonadotropin intermediated signaling.

All novel receptors are expressed in one or more reproductive tissues. Expression of SEQ ID NO:1 is observed in a limited number of tissues, including male but not female reproductive organs. SEQ ID NO:7 and SEQ ID NO:9 are extensions of SEQ ID NO:1. SEQ ID NO:3 is also expressed in a limited number of tissues but prominent expression is found in ovary. In contrast, SEQ ID NO:2 is expressed in the majority of tissues tested, including ovary, testis and uterus.

The identification of additional gonadotropin receptors could be a major step forward to the existing clinical therapies based on the existence of the known gonadotropin receptors as all gonadotropin mediated abnormalities and/or diseases are ascribed to these receptors. The receptors according to the invention will be useful in the development of hormone analogs that selectively activate either one of the classical gonadotropin receptors or the novel receptor according to the invention. This should be considered as one of the major advantages of the present invention. Alternatively, also analogs can be detected which inhibit the receptor function.

Thus, the invention will facilitate better treatment of fertility associated diseases and/or the design of new drugs for contraception.

Also functional equivalents that is receptors comprising SEQ ID NOs:4, 5, 6, 8, 10, 14, 15, 16 or parts thereof having variations of the sequence while still maintaining functional characteristics, are included in the invention.

The variations that can occur in a sequence may be demonstrated by (an) amino acid difference(s) in the overall sequence or by deletions, substitutions, insertions, inversions or additions of (an) amino acid(s) in said sequence. Amino acid substitutions that are expected not to essentially alter biological and immunological activities, have been described. Amino acid replacements between related amino acids or replacements which have occurred frequently in evolution are, inter alia Ser/Ala, Ser/Gly, Asp/Gly, Asp/Asn, Ile/Val (see Dayhof, M.D., Atlas of protein sequence and structure, Nat. Biomed. Res. Found., Washington D.C., 1978, vol. 5, suppl. 3). Based on this information Lipman and Pearson developed a method for rapid and sensitive protein comparison (Science, 1985, 227, 1435-1441) and determining the functional similarity between homologous polypeptides. It will be clear that also polynucleotides coding for such variants are part of the invention.

The polypeptides according to the present invention include the polypeptides comprising SEQ ID NOs:4, 5, 6, 8, 10, 14, 15 or 16 but also polypeptides with a similarity of 70%, preferably 90%, more preferably 95%. Also portions of such polypeptides still capable of conferring biological effects are included. Especially portions which still bind to ligands form part of the invention. Such portions may be functional per se, e.g. in solubilized form or they might be linked to other polypeptides, either by known biotechnological ways or by chemical synthesis, to obtain chimeric proteins. Such proteins might be useful as therapeutic agent by preventing the ligand from interacting with the natural gonadotropin receptors in the body.

Alternatively, downregulation of the expression level of the receptor can be obtained by using anti-sense nucleic acids through triple-helix formation (Cooney et al., 1988, Science, *241*, 456-459) or by binding to the mRNA. This in itself could also lead to treatment of infertility or to contraception.

A wide variety of host cell and cloning vehicle combinations may be usefully employed in cloning the nucleic acid sequence coding for the receptor the ligand-binding domain of the invention. For example, useful cloning vehicles may include chromosomal, non-chromosomal and synthetic DNA sequences such as various known bacterial plasmids and wider host range plasmids and vectors derived from combinations of plasmids and phage or virus DNA.

Vehicles for use in expression of the receptor or ligand-binding domain thereof of the present invention will further comprise control sequences operably linked to the nucleic acid sequence coding for the ligand-binding domain. Such control sequences generally comprise a promoter sequence and sequences which regulate and/or enhance expression levels. Of course control and other sequences can vary depending on the host cell selected.

Suitable expression vectors are for example bacterial or yeast plasmids, wide host range plasmids and vectors derived from combinations of plasmid and phage or virus DNA. Vectors derived from chromosomal DNA are also included. Furthermore an origin of replication and/or a dominant selection marker can be present in the vector according to the invention. The vectors according to the invention are suitable for transforming a host cell.

Recombinant expression vectors comprising the DNA of the invention as well as cells transformed with said DNA or said expression vector also form part of the present invention.

Suitable host cells according to the invention are bacterial host cells, yeast and other fungi, plant or animal host such as Chinese Hamster Ovary cells or monkey cells. Thus, a host cell which comprises the DNA or expression vector according to the invention is also within the scope of the invention. The engineered host cells can be cultured in conventional nutrient media which can be modified e.g. for appropriate selection, amplification or induction of transcription. The culture conditions such as temperature, pH, nutrients etc. are well known to those ordinary skilled in the art.

The techniques for the preparation of the DNA or the vector according to the invention as well as the transformation or transfection of a host cell with said DNA or vector are standard and well known in the art, see for instance Sambrook et al., *Molecular Cloning: A laboratory Manual*. 2^{nd} Ed., Cold Spring Harbor Laboratory, Cold Spring Harbor, NY, 1989.

The proteins according to the invention can be recovered and purified from recombinant cell cultures by common biochemical purification methods including ammonium sulfate precipitation, extraction, chromatography such as hydrophobic interaction chromatography, cation or anion exchange chromatography or affinity chromatography and high performance liquid chromatography. If necessary, also protein refolding steps can be included.

Gonadotropin hormone receptors according to the present invention can be used for the *in vitro* or *in vitro* identification of novel ligands or hormonal analogs. For this purpose binding studies can be performed with cells transformed with DNA according to the invention or an expression vector comprising DNA according to the invention, said cells expressing the gonadotropin receptor according to the invention.

Alternatively also the novel purified hormone receptor according to the invention as well as the ligand-binding domain thereof can be used in an assay for the identification of functional ligands or hormone analogs for the receptor.

Methods to determine receptor binding as well as *in vitro* and *in vivo* assays to determine biological activity of gonadotropins are well known. In general, expressed receptor is contacted with the compound to be tested and binding, stimulation or inhibition of a functional response is measured.

Thus, the present invention provides for a method for identifying ligands for gonadotropin receptors according to the invention, said method comprising the steps of:
- introducing into a suitable host cell a DNA or an expression vector according to the invention,
- culturing cells under conditions to allow expression of the DNA sequence
- optionally isolating the expression product
- bringing the expression product (or the host cell from the second step) into contact with potential ligands which will possibly bind to the ligand-binding domain of the receptor protein encoded by said DNA from the first step;
- establishing the amount of binding of the ligand to the receptor.

As an alternative to the binding of the ligand to the receptor also signal transduction capacity may be established.

SEQ ID NO:8 comprises the C-terminal part of the receptor, including all its transmembrane domains. This part is encoded by the nucleic acid of SEQ ID NO:7 from nucleotide 2-1487. This polypeptide part can also be used in a similar screenings assay to detect low molecular weight compounds by binding or by measuring the second messenger levels. Another part of this receptor is indicated in SEQ ID NO:4 and is encoded by SEQ ID NO:1. The complete sequence of this receptor is shown in SEQ ID NO:10 and is encoded by the nucleotides 1-2541 of SEQ ID NO:9. The polypeptide of SEQ ID NO:6 is encoded by SEQ ID NO:3. SEQ ID NO:5, SEQ ID NO:14, SEQ ID NO:15 and SEQ ID NO:16 all belong to the same receptor ad are encoded by SEQ ID NO:2, SEQ ID NO:11, SEQ ID NO:12 and SEQ ID NO:13, respectively.

The present invention thus provides for a quick and economic method to screen for therapeutic agents for the prevention and/or treatment of diseases related to reproductive tissues or for contraception. The method according to the invention furthermore provides for the selection of selective therapeutic agents discriminating between different gonadotropin receptors thus leading to a more effective therapeutic agent and/or diminishing of side effects. The method is especially suited to be used for the high through put screening of numerous potential compounds.

Compounds which activate or inhibit the receptor function may be employed in therapeutic treatments to activate or inhibit the receptors of the present invention.

Also within the scope of the invention are antibodies, especially monoclonal antibodies raised against the receptor molecule according to the invention. Such antibodies can be used therapeutically to inhibit receptor function and diagnostically to detect receptor molecules.

The invention furthermore relates to the use of the receptor genes as part of a diagnostic assay for detecting fertility abnormalities or susceptibility to infertility related to mutations in the nucleic acid sequences encoding these receptors. Such mutations may e.g. be detected by using PCR (Saiki et al., 1986, Nature, *324*, 163-166). Also the relative levels of RNA can be determined using e.g. hybridization or quantitative PCR technology. The presence and the levels of the receptors themselves can be assayed by immunological technologies such as radioimmuno assays, Western blots and ELISA using specific antibodies raised against the receptor. Such techniques for measuring RNA and protein levels are well known to the skilled artisan.

The determination of expression levels of different receptors in individual patients may lead to fine tuning of treatment protocols.

The following examples are illustrative for the invention and should in no way be interpreted as limiting the scope of the invention.

### Legends to the figures

### Figure 1

### Nothern blot of sequence SEQ ID NO:1

X-ray autoradiograph of a multiple tissue Nothern blot type II (Clontech) hybridized with a ³²P labeled probe obtained from SEQ ID NO:1. The lanes contain poly A⁺ RNA from the following human tissues/cell-types: spleen (1), thymus (2), prostate (3), testis (4), ovary (5), small intestine (6), colon (7) and peripheral blood leukocytes (8).

### Figure 2

### Nothern blot of sequence SEQ ID NO:2

X-ray autoradiograph of a multiple tissue Nothern blot type II and IV (Clontech) hybridized with a ³²P labeled probe obtained from SEQ ID NO:2. The lanes contain poly A⁺ RNA from the following human tissues/cell-types: spleen (1), thymus (2), prostate (3), testis (4), ovary (5), small intestine (6), colon (7), peripheral blood leukocytes (8) and uterus (9).

### Figure 3

### Nothern blot of sequence SEQ ID NO:3

X-ray autoradiograph of a multiple tissue Nothern blot type II (Clontech) hybridized with a ³²P labeled probe obtained from SEQ ID NO:3. The lanes contain poly A⁺ RNA from the following human tissues/cell-types: spleen (1), thymus (2), prostate (3), testis (4), ovary (5), small intestine (6), colon (7) and peripheral blood leukocytes (8).

### Examples

### Example 1

### Sequence identification

Using parts of the DNA sequence and/or the protein sequence of the human gonadotropin receptors and receptors from evolutionary distant organisms, we have screened several databases for the presence of related human (partial) cDNA sequences. The identified cDNA clones were obtained from several tissues and sequenced using an automatic sequencer. Three cDNA clones were obtained encoding parts of the trasmembrane domains of three novel human gonadotropin receptors. The sequences are shown in SEQ ID NOs: 1, 2, 3, 7, 9, and 13. SEQ ID NOs 1, 7 and 9 are overlapping sequences. SEQ ID NO:7 contains all transmembrane domains. SEQ ID NO:9 encodes the complete protein. SEQ ID NOs:2, 11, 12 and 13 are part of the same receptor.
The gonadotropin receptors form a well defined family in the very large superfamily of the G-protein coupled receptors. Within this superfamily especially the seven transmembrane domains are relatively good conserved. However, there are specific motif differences between different families. Motifs in the trasmembrane domains III and VI are usually very specific for a certain family, and therefore could indicate the probability that a candidate receptor is indeed a member of the family of gonadotropin receptors. Indeed, especially trasmembrane domains III is very conserved among the novel receptors comprising SEQ ID NO:2 and 3 and the human gonadotropin receptors. In addition, transmembrane domain VI is very conserved among the receptors comprising SEQ ID NO:1 and SEQ ID NO:2 and the human gonadotropin receptors. Furthermore, the domains III and VI are much less conserved between the novel receptors and human G-protein coupled receptors other than gonadotropin receptors. Thus, it is concluded that the three novel protein sequences are derived from novel gonadotropin receptors.

### Example 2

### Tissue distribution of the expression of gonadotropin receptor no.1

In order to analyze the expression of the receptor comprising SEQ ID NO:1 in different human tissues Northern blot analysis was performed. A human Multiple Tissue Northern blot type II was obtained from Clontech, which contains poly A⁺ RNA from the following human tissues/cell-types: spleen (lane 1), thymus (lane 2), prostate (lane 3), testis (lane 4), ovary (lane 5), small intestine (lane 6), colon (lane 7) and peripheral blood leukocytes (lane 8). Prehybridization was performed in hybridization solution (0.5 M Phosphate buffer pH 7.5, 7 % Sodium Dodecyl Sulfate (SDS), 1 mM EDTA) for 1 hour at 65 °C. For RNA detection, ³²P labeled DNA fragments were generated with an Oligolabeling kit (Pharmacia) using a 440 bp fragment from receptor sequence no. 1. This fragment starts at the 5' end of the coding sequence and stops at a Hind III site approximately 440 basepairs downstream (comprising nucleotide no 1 to 426 in SEQ ID NO:1). The ³²P labeled probes were hybridized to the filters for 16 hours at 65 °C. Subsequently, the filters were washed in solutions with decreasing salt concentrations up to 0.3 x SSC + 0.1% SDS at 65 °C and exposed to an X-ray film (X-Omat AR, Kodak).

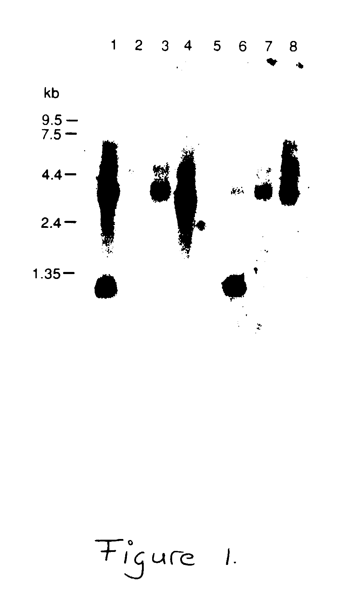

From the results shown in Figure 1 it can be concluded that gonadotropin receptor no. 1 is only expressed in a limited number of tissues, namely testis, prostate and spleen. Thus, expression of this receptor is observed in male but not in the female reproductive organs. The length of the mRNA in spleen and prostate is approximately 3.5-4.5 kb, while the length in testis is about 2.5-3.5 kb. In addition, a small mRNA of approximately 0.5-1.0 is observed in several tissues. However, the small length of this transcript suggests that it can not encode a full length gonadotropin receptor.

### Example 3

### Tissue distribution of the expression of gonadotropin receptor no.2

In order to analyze the expression of receptor no. 2 (comprising SEQ ID NO:2) in different human tissues Northern blot analysis was performed. Human Multiple Tissue Northern blots type II and IV were obtained from Clontech. Blot type IV is identical to blot type II (see example 2) with the exception that the poly A⁺ RNA obtained from ovary is replaced by poly A⁺ RNA from uterus (number 9). (Pre)hybridization and labeling of the probe was performed as described in example 2. For probe generation a 800 base pair fragment from receptor sequence no. 2 was used. This fragment starts at the 5' end of the coding sequence and stops at a Pvu II site approximately 800 basepairs downstream (comprising nucleotide no 1 to 664 in SEQ ID NO:2 plus in addition approximately 140 basepairs of unknown sequence). The filters were washed in solutions with decreasing salt concentrations up to 0.3 x SSC + 0.1% SDS at 65 °C and exposed to an X-ray film (X-Omat AR, Kodak).

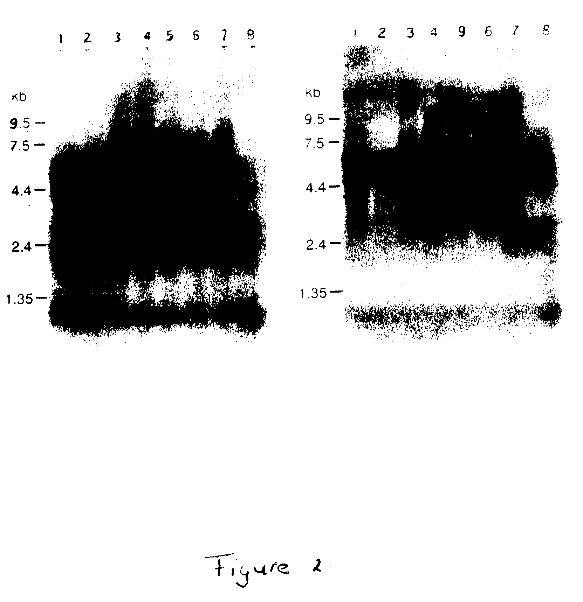

From the results shown in Figure 2 it can be concluded that gonadotropin receptor no. 2 is expressed in the majority of tissues tested, including ovary, testis and uterus. The length of the mRNA is approximately 5.5-6.5 kilobases.

### Example 4

### Tissue distribution of the expression gonadotropin receptor no.3

In order to analyze the expression of receptor no. 3 (comprising SEQ ID NO:3) in different human tissues Northern blot analysis was performed. The methods used are comparable to those described in example 2. As a probe a 820 base pair fragment from receptor sequence no. 3 was used. This fragment starts at the 5' end of the coding sequence and stops at a Not I restriction enzyme cleavage site (located immediately downstream of the DNA insert within the vector sequence) approximately 820 basepairs downstream (comprising nucleotide no 1 to 606 of SEQ ID NO:3 plus additional approximately 120 basepairs of unknown sequence). The filters were washed in solutions with decreasing salt concentrations up to 1 x SSC + 0.1% SDS at 65 °C and exposed to an X-ray film (BioMax, Kodak).

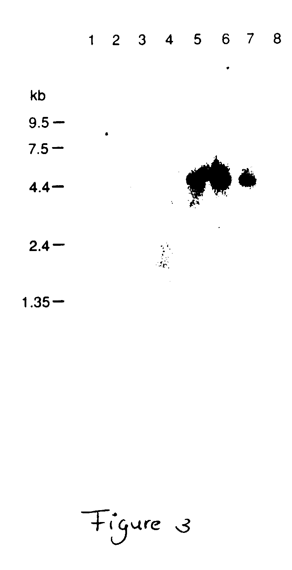

From the results shown in Figure 3 it can be concluded that gonadotropin receptor no. 3 is expressed in a limited number of tissues, including ovary. The length of the mRNA is approximately 5-6 kilobases.

## Claims

1. An isolated polynucleotide comprising one of the sequences selected from the group of polynucleotides encoding SEQ ID NO:4, SEQ ID NO:5, SEQ ID NO:6, SEQ ID NO:8, SEQ ID NO:10, SEQ ID NO:14, SEQ ID NO:15 or SEQ ID NO:16.

2. The polynucleotide according to claim 1, said polynucleotide comprising the sequences SEQ ID NO:1, SEQ ID NO:2, SEQ ID NO:3, SEQ ID NO:7, SEQ ID NO:9, SEQ ID NO:11, SEQ ID NO:12, SEQ ID NO:13 or the sequences extending from nucleotides 2-1487 from SEQ ID NO:7 or from nucleotides 1-2541 of SEQ ID NO:9.

3. A recombinant expression vector comprising the DNA according to claims 1 or 2.

4. Protein encoded by the polynucleotide according to claims 1 or 2 or the expression vector according to claim 3.

5. A cell transfected with DNA according to claims 1 or 2 or the expression vector according to claim 3.

6. A cell according to claim 5 which is a stable transfected cell which expresses the receptor protein according to claims 4.

7. Use of a DNA according to claims 1 or 2 or a expression vector according to claim 3, a cell according to claims 5 or 6 or a protein according to claim 4 in a screening assay for identification of new drugs.

8. A method for identifying ligands for the protein according to claim 4, said method comprising the steps of
• introducing into a suitable host cell a DNA according to claims 1, 2 or an expression vector according to claims 3;
• culturing the host cells under conditions to allow expression of the introduced DNA sequence;
• optionally isolating the expression product;
• bringing the host cell from the second step or the expression product from the third step into contact with potential ligands which will possibly bind to the ligand-binding domain of the expressed protein encoded by said DNA from the first step;
• establishing the amount of binding of the ligand to the expressed protein or its signal tranduction capacity.
